**Europäisches Patentamt**

**European Patent Office**

**Office européen des brevets**

(19)

(11) Veröffentlichungsnummer: **0 120 269**
**B1**

(12)

# EUROPÄISCHE PATENTSCHRIFT

(45) Veröffentlichungstag der Patentschrift:
**13.05.87**

(51) Int. Cl.⁴: **C 07 C 29/80**, C 07 C 31/04

(21) Anmeldenummer: **84101606.6**

(22) Anmeldetag: **16.02.84**

(54) **Verfahren zur Erzeugung von Methanol in Chemie-Qualität.**

(30) Priorität: **29.03.83 DE 3311316**

(43) Veröffentlichungstag der Anmeldung:
**03.10.84 Patentblatt 84/40**

(45) Bekanntmachung des Hinweises auf die Patenterteilung:
**13.05.87 Patentblatt 87/20**

(84) Benannte Vertragsstaaten:
**DE FR GB IT NL**

(56) Entgegenhaltungen:
**EP - A - 0 040 481**

(73) Patentinhaber: **Uhde GmbH, Friedrich-Uhde-Strasse 15, D-4600 Dortmund 1 (DE)**

(72) Erfinder: **Ilgner, Hartmut, Dipl.-Ing., Am Siepenhol 14, D-4600 Dortmund 30 (DE)**
Erfinder: **Thiagarajan, Natarajan, Dr.-Ing., Boemckestrasse 25, D-4600 Dortmund 1 (DE)**
Erfinder: **Heck, Günter, Dipl.-Ing., Buchenweg 1, D-6200 Wiesbaden 1 (DE)**
Erfinder: **Lienerth, Aladar, Dr. Dipl.-Ing., Am Steinbruch 6, D-6233 Kelkheim (DE)**

## Beschreibung

Die Erfindung betrifft ein Verfahren zur Erzeugung von Methanol in Chemie-Qualität durch Destillation von Rohmethanol in mehreren Destillationsstufen mit Trennung in Methanol in Chemie-Qualität Abgas und Fuselöl.

Die industrielle Methanolsynthese liefert ein Produkt, das nicht der Chemie-Qualität, auch «Grade AA» genannt, entspricht. Es ist daher erforderlich, das Produkt aus der Methanolsynthese destillativ aufzuarbeiten.

Es ist bekannt, die destillative Aufarbeitung des Rohmethanols in zwei Stufen vorzunehmen. In der ersten Stufe, auch «Topping Teil» genannt, werden die leicht siedenden Bestandteile, d.h. leichter als Methanol, abgetrennt und als Abgas einer weiteren Verwendung zugeführt. In der zweiten Stufe, die sowohl eine als auch mehrere Destillationskolonnen aufweisen kann und «Refining Teil» genannt wird, werden die schwersiedenden Bestandteile, d.h. schwerer als Methanol, so z.B. Wasser abgetrennt. Diese Abtrennung ist jedoch mit dem Problem der Änderung des Konzentrationsprofils Methanol/Äthanol/Wasser/Druck behaftet. Um die erforderliche Methanol-Qualität von u.a. einem Restäthanolgehalt von max. 10 Gew.-ppm erreichen zu können, ist ein Seitenabzug im Abtriebsteil jeder Destillationskolonne erforderlich. Der Seitenabzug, bekannt unter dem Namen «Fuselöl», ist ein Gemisch aus Methanol, Wasser und höheren Alkoholen wie Äthanol, Propanol u.a. Der Methanolgehalt des Fuselöls ist so hoch, dass bezogen auf den Methanolgehalt im Rohmethanol ca. 1,5% der Methanolproduktion verloren ist.

Aus EP-A-40481 ist ein Methanol-Erzeugungsprozess bekannt, bei dem ausgehend von der Synthesegas-Produktion aus Erdgas letztendlich durch mehrstufige Destillation Methanol in Chemie-Qualität gewonnen wird. Der abgetrennte Fuselölstrom mit mehr als 50% Methanol wird in das Einsatzerdgas zurückgeführt und vermindert die Methanolausbeute auf max. 98,5%. Die Fuselöl-Rückführung bedingt Vergrösserungen der Anlagenkomponenten und damit erhöhten Energiebedarf in der Destillation.

Der Erfindung liegt die Aufgabe zugrunde, den bisherigen Methanol-Verlust im Fuselöl zu reduzieren.

Diese Aufgabe wird erfindungsgemäss dadurch gelöst, dass ein Verfahren gemäss dem Kennzeichen des Patentanspruchs angewendet wird.

Mit dem erfindungsgemässen Verfahren wird erreicht, dass der Methanolverlust von bisher 1,5% reduziert wird auf nur 0,2% des Methanols im Rohmethanol aus der Methanolsynthese. Der dazu erforderliche Energieaufwand steht in keinem Verhältnis zum Produktgewinn. Bei einer Methanolsyntheseanlage mit einer Tagesleistung von z.B. 2000 t beträgt der Gewinn damit ca. 30 t pro Tag. Durch die Entnahme des Rücklaufproduktes für die Fuselöl-Destillationsstufe aus der letzten Methanol-Destillationsstufe resultiert ein spezifisch geringerer Energiebedarf.

Ein Ausführungsbeispiel der Erfindung ist in der Zeichnung dargestellt und wird im folgenden näher beschrieben. Es zeigen:

Fig. 1 Verfahrensschema einer Rohmethanol-Destillation;

Fig. 2a Diagramm Methanol/Äthanol in Abhängigkeit vom Druck;

Fig. 2b Diagramm Methanol/Äthanol in der Abtriebssäule.

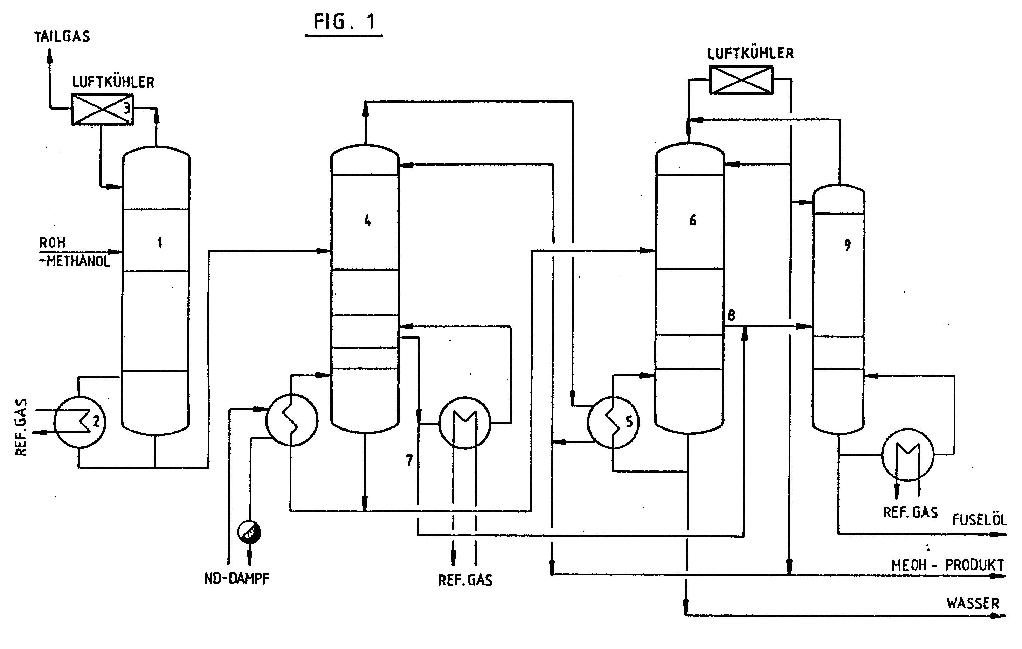

Nach Fig. 1 wird das Rohmethanol der «Topping-Kolonne» 1 in bekannter Weise mittig zugeführt. Über den Wärmetauscher 2 erfolgt die Aufheizung des Rohmethanols. Die leichter als Methanol siedenden Bestandteile verlassen als Abtrieb den Kopf der Kolonne, werden in einem Kondensatteil 3 von mitgerissenem Methanol getrennt und stehen für eine weitere Verarbeitung zur Verfügung. Das so vorgereinigte Rohmethanol aus dem Sumpf der «Topping-Kolonne» wird der «Refining-Stufe» I mit der Kolonne 4 zugeführt. Das Kopfprodukt aus dieser Kolonne 4 heizt den Aufkocher 5 der Kolonne 6 der «Refining-Stufe» II, in welche das Sumpfprodukt der Kolonne 4 zur weiteren Destillation führt.

Das Kondensat aus dem Aufkocher 5 ist teilweise Rücklauf und teilweise Reinmethanol des Reinheitsgrades AA. Über Leitung 7 wird der Kolonne 4 ein Abtriebsstrom, sog. Fuselöl entzogen.

Das Kopfprodukt der Kolonne 6 wird in bekannter Weise kondensiert und als Rücklauf abgezogen und als Reinmethanol gewonnen. Das Sumpfprodukt ist im wesentlichen Wasser, welches einer weiteren Verwertung zugeführt werden kann. Das auch aus der Kolonne 6 über die Leitung 8 abgezogene Fuselöl aus dem Abtriebsteil der Kolonne 6 wird mit dem Fuselöl in Leitung 7 zusammengeführt und der Methanolrückgewinnungsanlage 9 zugeführt. Infolge des hohen Rücklaufverhältnisses in dieser Anlage kommt es zu einer guten Trennwirkung in bezug auf das vorhandene Restmethanol. Das abgezogene Methanol hat den Reinheitsgrad AA und das als Sumpfprodukt anfallende Fuselöl wird zur weiteren Verwendung abgeführt.

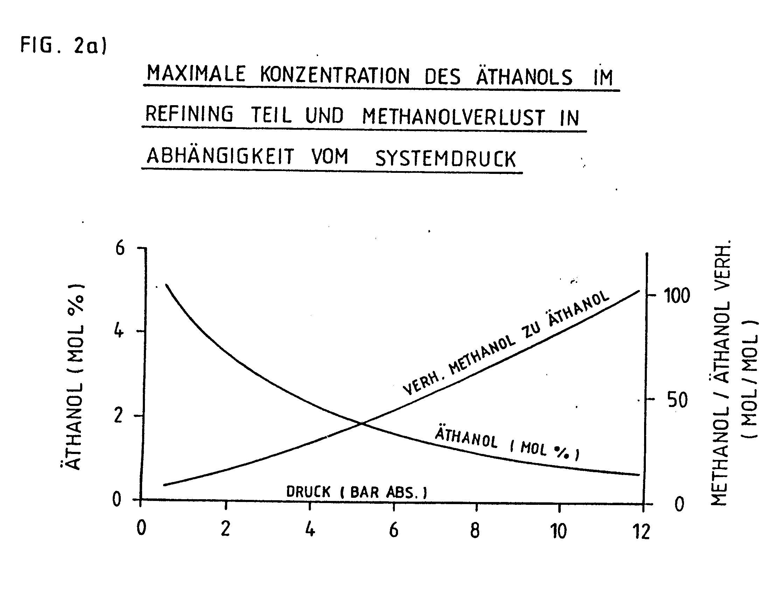

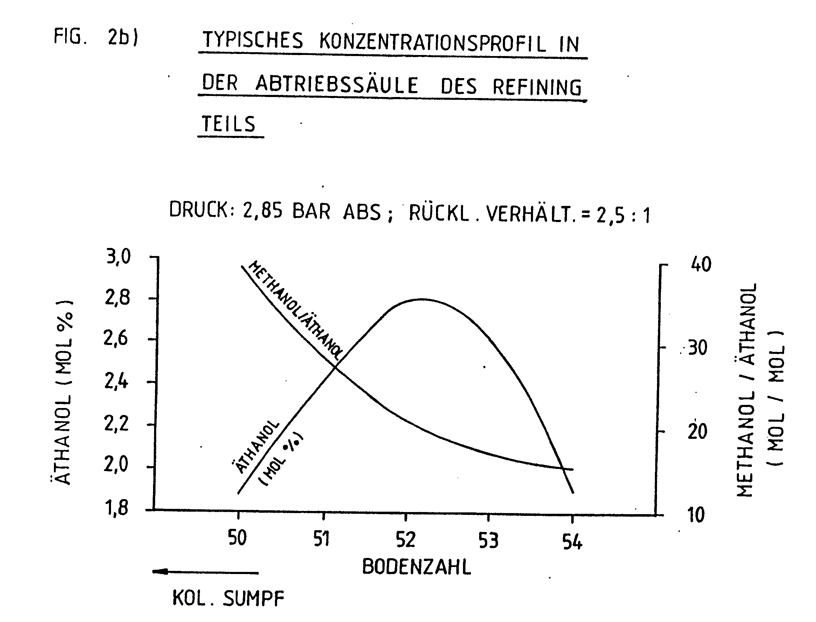

Aus den Diagrammen der Fig. 2a und 2b sind die Abhängigkeiten der Gemische zu erkennen. So ergibt sich z.B. aus Diagramm 2a bei einem Systemdruck von 2,85 bar abs. ein Äthanolgehalt von 2,8 Mol-% und ein Methanol/Äthanol-Verhältnis von 22:1.

Fig. 2b zeigt, dass in der Abtriebssäule am 52. Boden das Konzentrationsmaximum des Äthanols liegt und zwar bei einem verhältnismässig niedrigeren Methanol/Äthanol-Verhältnis.

## Patentanspruch

Verfahren zur Erzeugung von Methanol in Chemie-Qualität durch Destillation von Rohmethanol in mehreren Destillationsstufen mit Trennung in Methanol in Chemie-Qualität Abgas und Fuselöl, dadurch gekennzeichnet, dass das abgezogene Fuselöl in einer zusätzlichen Destillationsstufe bei einem Rücklaufverhältnis von 5:1 und höher destilliert wird, wobei das Rücklaufprodukt für die Fuselöl-Destillationsstufe der letzten Methanol-Destillationsstufe entnommen wird.

**Revendication**

Procédé de production de méthanol de qualité chimique par la distillation du méthanol brut dans plusieurs étages de distillation, avec séparation du méthanol de qualité chimique, du gaz du queue et de l'huile de fusel, caractérisé en ce que l'huile de fusel soutirée est distillée dans un autre étage de distillation, le rapport de retour étant de 5:1 et plus, étant entendu que le produit de retour destiné à l'étage de distillation de l'huile de fusel est prélevé dans le dernier étage de distillation du méthanol.

**Claim**

Process for the production of grade AA methanol by distilling raw methanol in several distillation stages, thereby separating grade AA methanol, tail gas, and fusel oil, characterized in that the fusel oil withdrawn is distilled in an additional distillation stage at a return ratio of 5:1 or higher, the return product required for the fusel oil distillation stage being withdrawn from the last methanol distillation stage.

FIG. 1

LUFTKÜHLER

TAILGAS

LUFTKÜHLER

ROH-METHANOL

REF. GAS

ND-DAMPF

REF. GAS

REF. GAS  FUSELÖL

MEOH - PRODUKT

WASSER

FIG. 2a)

MAXIMALE KONZENTRATION DES ÄTHANOLS IM

REFINING TEIL UND METHANOLVERLUST IN

ABHÄNGIGKEIT VOM SYSTEMDRUCK

Y-axis (left): ÄTHANOL (MOL %): 0, 2, 4, 6
Y-axis (right): METHANOL / ÄTHANOL VERH. (MOL / MOL): 0, 50, 100
X-axis: DRUCK (BAR ABS.): 0, 2, 4, 6, 8, 10, 12
VERH. METHANOL ZU ÄTHANOL
ÄTHANOL ( MOL % )

7

FIG. 2b) <u>TYPISCHES KONZENTRATIONSPROFIL IN</u>

<u>DER ABTRIEBSSÄULE DES REFINING</u>

<u>TEILS</u>

DRUCK: 2,85 BAR ABS ; RÜCKL. VERHÄLT. = 2,5 : 1

KOL. SUMPF